# EUROPEAN PATENT APPLICATION

(11) **EP 4 361 280 A1**
(43) Date of publication of application: **01.05.2024**
(21) Application number: 21947288.3
(22) Date of filing: 23.06.2021
(51) Int. Cl.: C12P 1/02, A23L 3/3571, A61K 38/43

(54) **FOOD IN CONTROLLED DYNAMIC FERMENTATION FOR BACTERIOSTASIS OF BENEFICIAL MICROORGANISMS, KEEPING THEM ALIVE AND METABOLICALLY ACTIVE FOR HUMAN CONSUMPTION**

(71) Applicant: Kuragobiotek Holdings S. A. P. I. De C.v., Ciudad del Sol Zapopan, Jalisco, 45050 (MX)
(72) Inventor: CRUZ SERRANO, José Antonio, El Salto, Jalisco, 45693 (MX)
(74) Representative: Potter Clarkson
(86) International application number: PCT/MX2021/000021
(87) International publication number: WO 2022/271002

(57) **Abstract**

The present invention relates to a dynamic fermentation in a food matrix that generates bacteriostasis in probiotic microorganisms, enabling them to be kept alive and metabolically active, that is, converting nutrients into secondary metabolites such as proteins, acids, enzymes and even more so biomass as a product of their biological activity, and maintaining safety as a result of pathogen antagonism and competition with regard to other unwanted microorganisms in the food matrix due to a balance in the physical parameters (temperature, viscosity, presence of water, osmotic pressure, lighting level and electrical conductivity), the chemical parameters (hydrogen potential, concentration of solids, colloid phase and available oxygen levels), the nutritional parameters (in terms of the presence or absence of proteins, short-chain fatty acids, carbohydrates, minerals and vitamins), as well as biological parameters in concentrations of colony-forming living cell units of live beneficial and metabolically active microorganisms which can be either anaerobic and aerobic.

## Description

### TECHNICAL FIELD

The technical field of the present invention is based in the biotechnology area, providing a method for obtaining a food in controlled dynamic fermentation prepared with all food grade ingredients, whose objective is to achieve a balance among the following physical parameters: temperature, viscosity, presence of water, osmotic pressure, lighting level and electrical conductivity; chemical parameters, such as hydrogen potential, concentration of solids, colloid phase and available oxygen levels; nutritional parameters, in terms of the presence or absence of proteins, short-chain fatty acids, carbohydrates, minerals and vitamins, as well as biological parameters in concentrations of colony-forming living cells units of beneficial and metabolically active microorganisms which can be either anaerobic and aerobic. Said balance among above mentioned parameters allows to generate a bacteriostasis in the group of beneficial and metabolically active microorganisms. This bacteriostasis creates a controlled and dynamic fermentation over time, leading to a shelf life of up to 8 months, even at ambient temperature, in nonperforming microorganisms by keeping food safety in the fermented food, free and clear of any pathogenic microorganism, but maintaining the living presence and metabolically active of beneficial inoculated microorganisms that originally fermented the food, without any loss greater than 2 decimal logarithms in the concentration of their colony-forming units and maintaining food nutrients intact, both those constituting the food ingredients itself and the secondary metabolites generated in the fermentation process, specifically, acids, enzymes and proteins, which, as a whole, allow for nutrition in human consumption, with no limitation to animal nutrition.

*Object of the invention.* - The object of this invention is to produce bacteriostasis in probiotic microorganisms that holds harmless from pathogenic microorganisms by pathogenic antagonism, increases the shelf time even at ambient temperature for up to 8 months of a food in dynamic fermentation by establishing a balance among physical parameters (such as temperature, viscosity, presence of water, osmotic pressure, lighting level and electrical conductivity); chemical parameters (hydrogen potential, concentration of solids, colloid phase and oxygen levels; nutritional parameters in terms of the presence and absence of proteins, short-chain fatty acids, carbohydrates, minerals and vitamins, as well as biological parameters in concentrations of colony-forming living cells of beneficial and metabolically active microorganisms, while maintaining the integrity of all nutrients that constitute the food, and allowing it for human consumption, with no limitation to animal nutrition.

### BACKGROUND

Scientific publication "Probiotic fermented foods for health benefits" by Dr. Beena Divya, with registry 12, No. 4, 377-390 2012, published by the Biotechnology Division of the National Institute for Interdisciplinary Science and Technology (NIIST), CSIR, Trivandrum, Kerala, India. It provides the consideration for several factors that may influence probiotic viability in foods, as well as their survival when entering human gastrointestinal tract. It is restricted to see fermentation as an improvement of flavor and nutritional quality of foods and their shelf life as compared with their natural degradation process. The publication mentions that fermentation products include organic acids (e.g. palmitic, pyruvic, lactic, acetic, propionic and butyric acid); alcohols (mainly ethanol), aldehydes and ketones. It also makes reference to safe metabolic activity, and that homofermentative bacteria generate tow moles of lactate per mole of glucose, while heterofermentative bacteria use the pentose phosphate pathway to produce equimolar quantities of lactate, CO2 and ethanol from glucose. It exemplifies how to use a small amount of a batch previously fermented to inoculate a new batch. It compares traditional fermentation versus commercial fermentation, a native mixed culture versus a limited biodiversity in commercial cases. It worth mentioning the reference to bacteriocins of less than 20 kDa that cause membrane depolarization of the target cell and/or inhibit the cell wall synthesis and those with more than 20 kDa that degrade the murein layer. It considers several factors that may influence the viability of the culture, as well as its activation in the intestine. These factors include (i) the physiological state of probiotic organisms added in the growth phase, (ii) storage conditions, for example, temperature, humidity, (iii) chemical composition of the food matrix, for example, tithable acidity, available content of carbohydrates, nitrogen sources, vitamins, minerals, prebiotic, food additives, water activity and content of oxygen, and (iv) possible interaction between starting probiotics and cultures, for example, antagonism. It establishes methods to preserve sensible biological materials, including lyophilization, cryopreservation and spray drying, where it must be * 107 UFC/g over the minimum viable concentration. Lastly, the article mentions the Technische Universität München (TUM) *and* how they developed an environment-friendly vacuum system at low temperature to increase the cellular viability rate. It concludes that probiotic bacteria microencapsulation using several biopolymers may significantly increase viability. In every case, the publication cannot establish a break-even point among the physical, chemical, nutritional and biological parameters that may cause a bacteriostasis among each other in the original beneficial microorganisms inoculated for dynamic fermentation by keeping them alive and metabolically active for up to 8 months in the shelf, even at room temperature, being free and clear of any pathogenic microorganism and maintaining the food integrity that allows it for human consumption, with no limitation to animal nutrition.

Patent EP 2 206 560 A1. The probiotic formulation dated December 18, 2008 states probiotic compositions and kits formed by living bacteria that belong to the human body natural flora, non proteinaceous chelators with low molecular weight capable of reducing iron concentration throughout the entire range of physiological pH relevant to the levels that inhibit the growth of pathogens, but allow for the bacteria growth of the composition, therapeutic formulations, non-protein iron chelators with low molecular weight, treatment of infections in the human body cavities. The therapy against infections that resist antibiotics in the body cavities, vaginal tract, male urethra, intestine and oral cavity describes that they are naturally colonized by probiotic bacteria, both anaerobic and aerobic ones, with preventive or even healing properties of diseases competing for the union, inhibition of pathogenic growth, presented in vaginal capsules comprising a variant of Lactobacillus, vaginal suppository, vaginal medicines, pharmaceutical formulations, fermented dairy products. It establishes how the iron is an essential growth factor to almost every cells and microorganisms, the lactoferrin (see The Merck Index, XIII Ed., 2001, No. 9647), a glycoprotein endogenously produced by neutrophils and also known for being a main component of the secreted fluids, including saliva, gastric juice and bile, a very important factor in the bacteriostatic system of the breast milk with a purity of up to 95% and in amounts up to 480mg. Some combinations of lactic acid bacteria with lactoferrin are also commercially available (for example, colostrum with lactoferrin chewable tablets, Peak Nutrition Inc., Syracuse NY) in this product, however, the amount of living bacteria (3.4 × 106 UFC) do not present clinical studies or in animals that prove the efficacy of combining lactic acid bacteria with lactoferrin over bacteria alone. Commensal lactobacilli may increase the availability of iron for gonorrhea, where chelators are intended for their bactericide or even sterilizing property. There is no a description of products that combine lactic acid bacteria or bifidobacterium with non-protein iron chelators with low molecular weight, which unexpectedly cause the probiotic bacteria growth while inhibiting pathogenic microorganism growth, but it does describe a pharmaceutical or probiotic composition formed by (a) at least one species and a variant of lactobacillus or at least one species and a variant of bifidobacterium, or mixtures thereof, and (b) at least one non protein iron chelator with low molecular weight, species and variants that have good tolerance in humans and affinity by human mucous, 10kDa chelators, preferably lower than 5kDa and even more preferably lower than 1kDa, which is far below the typical molecular weight (MW) associated to protein structures (for example, lactoferrin MW = 80kDa), calcium and neutral salts complexes, oral or vaginal administration, drink, one capsule, one infant formula or even as a dairy product similar to yogurt, cheese, ice cream, a product based on fermented cereals, a milk powder, an infant formula, a tablet, a capsule, a liquid suspension, a powder or dry oral sand, a paste or oral humid jelly, a powder or sand intended for tube feeding or a liquid for humid tube feeding. Alternatively, the drink may be prepared before use from a soluble capsule containing the active ingredients, preferably, the drink may be prepared before use reconstituting a powder that contains lyophilized bacteria and iron chelator or, alternatively, reconstituting a dry powder that contains lyophilized bacteria with a physiological solution including the chelator; the dry powder is preferably packed in hermetically sealed packets protected from air and light, under air or nitrogen, under a noble gas or including vacuum, with additional excipient, inulin, fructose, starch, xylooligosaccharides, silicon oxide, buffering agents and flavors. In every case, the patent cannot establish a break-even point among the physical, chemical, nutritional and biological parameters that may cause a bacteriostasis among each other in the original beneficial microorganisms inoculated for dynamic fermentation by keeping them alive and metabolically active for up to 8 months in the shelf, even at room temperature, being free and clear of any pathogenic microorganism and maintaining the food integrity that allows it for human consumption, with no limitation to animal nutrition.

Patent US 6,645,515 B1, bacteriostatic composition for salmonella from Nov. 25, 1999 by Meito Sanyo Kabushiki Kaisha, states a bacteriostatic composition for salmonella, an additive, medicine or healthy foods for the prophylaxis or treatment of salmonellosis, preventing this by the administration of antibiotics, vaccines, Streptococcus or Lactobacillus, generating a competitive exclusion of salmonella, as a proven healthy food. It mentions how antibiotics have difficulties with resistant variants, how vaccines have difficulties since they are only effective in particular pathogens, the oligosaccharides are not effective on residual pathogenic salmonella, however, oligosaccharides promote the growth of forked bacteria, while some viable antibiotics, vaccines, and cellular agents set pathogens adding and inhibiting their adhesion. The inhibiting effect of the salmonella, prophylaxis or treatment of salmonella in preparations for infections originated in a fermented broth obtained by *Leuconostoc, Streptococcus* and *Streptobacterium* in a nutrient containing sucrose, medium fermented broth as active ingredient, to prepare a composition for the prophylaxis or treatment of salmonellosis in animals, ferment produced by microorganisms *Leuconostoc mesenteroides, Leuconostoc dextranicum, Streptococcus bovis, Streptococcus* mutans, *Streptococcus sanguis* and *Streptobacterium dextranicum,* the nutritional medium containing sucrose to ferment the bacteria contains, as a source of carbohydrates, sucrose, sucrose in isolated form or the residue extracted from the sugar cane or sugar/molasses, beetroot pulp, husk, brewer's yeast extract, peptone, corn liquor, soy protein, table salt, HCI (hydrochloric acid) K2HP04 (dipotassium phosphate). Dipotassium phosphate (K2HPO4) (also dipotassium hydrogen orthophosphate; potassium phosphate dibasic) is an inorganic compound at 25 C in anaerobic conditions, the typical fermented broth or a concentrate of the supernadent, coming from the fermented broth that is already free from living cells. The product is spray drying or lyophilized, fraction by precipitation. Ethanol, methanol, acetone and isopropyl alcohol are referred to remove cells from the fermented broth, before or after treatment of fractioned precipitation or simultaneously, cell removal may be performed through filtering or centrifugation. Said supernadent may also achieve the desire object of the invention. The embodiment of the invention may be a liquid medicine that is obtained by simply dissolving above-mentioned preparation in a liquid or syrup (concentrate), granulation agent or solidification commonly used in the pharmaceutical or food industries, lyophilizing or spray drying the liquid or syrup preparation, or mixing already the prepared dry material, produced based on the bacteria and, therefore, a fermented broth that comprises several ingredients that may probably contain lactic acid, fructose, mannitol, leucrose, cells of the lactic acid bacteria used for fermentation, reduces excrement pH and has an excellent effect on the production of chicken eggs, cow meat and milk, without salmonella. The rate of use of the preparation contained as active ingredient in the bacteriostatic composition for salmonella used in poultry, such as chickens and turkeys, pets, cattle, such as horses and pigs, with no limitation to humans. In every case, the patent cannot establish a break-even point among the physical, chemical, nutritional and biological parameters that may cause a bacteriostasis among each other in the original beneficial microorganisms inoculated for dynamic fermentation by keeping them alive and metabolically active for up to 8 months in the shelf, even at room temperature, being free and clear of any pathogenic microorganism and maintaining the food integrity that allows it for human consumption, with no limitation to animal nutrition.

Patent US 6,172,040 BE, lactoferrin antimicrobial activity immobilizer and its use, May 28, 1999 by A. Satyanarayan Naidu, describes a method to treat meat products, with immobilized lactoferrin, or reduce pathogenic microbial contamination using lactoferrin (LF) as immobilizer agent on a naturally occurring substrate, preferably a galactose rich polysaccharide; in some embodiments, the lactoferrin is applied as an aqueous solution containing a mixture of immobilized lactoferrin and native lactoferrin, and a system that includes a physiologically acceptable acid, such as citric acid, a physiologically acceptable base, such as sodium bicarbonate, and a physiologically acceptable salt, such as sodium chloride, chemical agents and antimicrobial agents. It determines that healthy meat animals are essentially sterile, but when they are sacrificed, the animal is colonized, proliferating microbial contamination, such as verotoxigenic *Escherichia coli* (E. Coli), E. coli serotype 0157: H7, *Enterococcus faeciumi* strains, all of them can survive acid conditions, and irradiation is an efficient method to kill some types of E. coli. Lactic acid metabolites are naturally occurring substances that block microbial adherence-colonization, slow down growth-multiplication and neutralize adverse effects of the proinflammatory cells from residues; the degraded or denatured LF molecule generates fragments of cationic peptides, these cationic peptides show unspecific antimicrobial activity.

The invention refers to a method to treat products with a sufficient amount of lactoferrin to reduce microbial contamination and the immobilized lactoferrin used in the process; the lactoferrin is immobilized on a naturally occurring substrate through the N-terminal region of the lactoferrin. Adequate substrates include proteins, polysaccharides, cellulose nucleic acids, nucleotides and lipids; preferred substrates include collagen, gelatin, fibronectin, casein, mucin, heparan-sulfate, carrageenan, deoxyribonucleic acid, adenosine triphosphate or a triglyceride, being the galactose rich polysaccharide the most preferred one. It establishes that the system containing a physiologically acceptable acid, such as oxalic acid, acetic acid and citric acid, preferably citric acid, a physiologically acceptable base, preferably sodium bicarbonate, and a physiologically acceptable salt, such as calcium chloride, potassium chloride and sodium chloride, creating a method to reduce the microbial contamination of meat products. In every case, the patent cannot establish a break-even point among physical, chemical, nutritional and biological parameters that may cause a bacteriostasis among each other in the original beneficial microorganisms inoculated for dynamic fermentation, by keeping them alive and metabolically active for up to 8 months in the shelf, event at room temperature, being free and clear of any pathogenic microorganism and maintaining the food integrity that allows it for human consumption, with no limitation to animal nutrition.

### DETAILED DESCRIPTION OF THE INVENTION

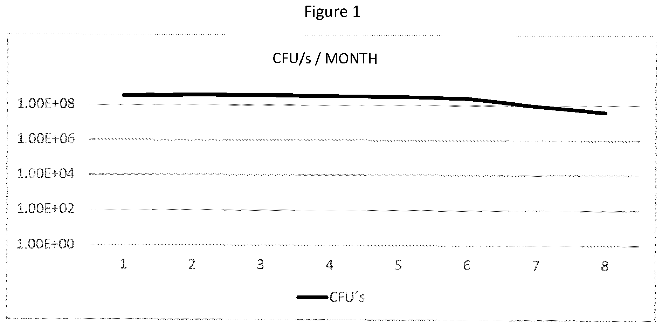

Characteristic details of this method to obtain a bacteriostasis in probiotic microorganisms that holds harmless from pathogenic microorganisms by pathogenic antagonism, increases the shelf time even at room temperature for up to 8 months of a food in dynamic fermentation through a balance of physical, chemical and nutritional parameters, keeping food integrity for human nutrition, with no limitation to animal nutrition, are clearly shown in the following description and the accompanying drawing, as well as the illustration of said method, and following the same reference indications to reference the figure.

The process described herein requires, due to its biological nature in food ferments, a 1,000 Class clean room with a maximum of 1,000 particles per cubic feet, whose size is equal and/or higher than 5 microns, ISO equivalent to 07, according to US FED STD 209 E, this environment shall be maintained during the entire process.

It is based on an initial controlled fermentation of a biological process where ingredients are deionized distilled water, glucose, agave inulin with a molecular structure with beta 2-1 and beta 2-6 binds resistant to hydrolysis by human digestive enzymes, but that can be fermented by native lactic acid bacteria from the human gastrointestinal tract, protein with complete aminogram in essential amino acids and the following profile:

### Content in edible essential amino acids (mg/L)

**Table 1. Amino acids**

| Amino acids | Mg/L | Range |
|---|---|---|
| Isoleucine | 5.30 | +/-0.795 |
| Leucine | 8.70 | +/-1.305 |
| Lysine | 5.70 | +/-0.855 |
| Methionine and cysteine | 3.30 | +/-0.495 |
| Phenylalanin e and tyrosine | 19.00 | +/-2.850 |
| Threonine | 4.00 | +/-0.600 |
| Tryptophan | 0.60 | +/-0.090 |
| Valine | 5.10 | +/-0.765 |

Brewer's yeast, typically *Saccharomyces cerevisiae* or any other used in the bakery or brewing industry, and food grade salt compositions as described below.

### Food grade salt composition (^{%})

**Table 2. Food grade salts**

| Salt (food grade) | % |
|---|---|
| Sodium acetate, anhydrous | 54.35% |
| Potassium phosphate, dibasic | 21.74% |
| Dibasic ammonium citrate | 21.74% |
| Magnesium sulfate | 2.17% |
| TOTAL | 100.00% |

A first mixture of carbohydrates is prepared by dissolving 100 +/- 10g of glucose and 25 +/-2.5g of agave inulin in 1.25 +/- 0.1875L of deionized distilled water with a molecular structure with beta 2-1 and beta 2-6 binds. A second protein mixture is prepared by dissolving 75 +/-3.25g of protein with the content of edible essential amino acids (mg/L) described in Table 1, in 2.25 +/- 0.1L of deionized distilled water. A third mixture is prepared with 57.5 +/- 2.875g of a food grade salt composition using the proportions indicated in Table 2, adding 50 +/-2.50g of protein into the content of edible essential amino acids (mg/L) described in Table 1, 25 +/- 1.25g of brewer's yeast, typically *Saccharomyces cerevisiae* or any other used in the bakery or brewing industry. The solid bases of the third mixture are combined into 3 +/- 0.3L of deionized distilled water.

These mixtures shall feed a bioreactor for the culture of probiotic strains, both individually or in synergistic consortium, which could be the following, with no limitation to other that fulfill the condition of being lactic acid bacteria or yeasts native from the human gastrointestinal tract.

Those probiotic strains are:

| **Lactobacill us** | **Bifid us** | **Streptococc us** | **Yeast** |
|---|---|---|---|
| *L. casei* | *B. breve* | *S. sanguis* | *Saccharomyc es boulardii* |
| *L. rhamsnosus* | *B. infantis* | *S. Bovis* | |
| *L. paracasei* | *B. bifidum* | *S. Cremorisir* | |
| *L. rauteri* | *B. bacterium* | *S. Thermophilus* | |
| *L. lactis* | *B. Lactis* | *S. gallalytius* | |
| *L. Fermentum* | *B. animalis* | | |
| *L. gasseri* | *B. suis* | | |
| *L. jahnsonii* | *B. longum* | | |
| *L. acidopillus* | | | |
| *L. cripanus* | | | |
| *L. amylovorus* | | | |
| *L. delbrueckii* | | | |
| *L. bulgaricas* | | | |
| *L. Plantarum* | | | |

Probiotics may be integrated in their lyophilized format, reconstituting them in the first mixture of carbohydrates at a temperature of 25 +/- 2°C. It is recommended to use the culture in the individual strain bioreactor or in synergistic consortium among all of the strains that form it, typically using a solid base. The probiotic lyophilized powder or synergistic consortium of probiotics have a concentration of 1×10₁₁ CFU per gram of dry base with 0.250 +/- 0.025L of mixture 1 of carbohydrates. This will create a concentration in the solution of about 5×10₁₁ and 1×10₁₂ CFU.

Once all the mixtures have been prepared for bioreaction, the bioreactor must have capacity for at least 10L and to maintain operative, pressure, and mechanical agitation speed parameters under control, which is preferably performed using a double-blade agitator at the end of the propulsion motor shaft located in the lower part of the bioreactor container, at an angle of 50 +/- 5 degrees,
temperature, hydrogen potential or acidity and time, within the ranges of these parameters shown in Table 3.

**Table 3. Bioreaction Parameters**

| Operatio n Paramet er | Unit | Magnit ude | Operati on Range |
|---|---|---|---|
| Pressure | Lb./in ² | 15 | +/-2 |
| Agitatio n Speed | RPM | 90 | +/- 10 |
| Temperat ure | °C | 37.5 | +/-2 |
| pH | 0-14 scale | 5.7 | +/- 0.4 |
| Time | Hours | 15 | +/- 5 |

These operation parameters shall be maintained under control throughout the bioreaction fermentation period, which begins when carbohydrate, protein and food grade stabilizing salts are entered through the supply port of the bioreactor, which is previously sterilized. These mixtures are left for 20 +/- 5 minutes for homogenization purposes, and then a gas is injected through the corresponding port to generate a controlled atmosphere with nitrogen with the following parameters: 15 +/- 2Lb/in² of pressure, 37.5 +/- 2° of temperature, and agitation speed of 90 +/- 10 revolutions per minute (RPM). pH shall be maintained under control at 5.7 +/- 0.4, by compensatory injection through the electronic control loop of a peristaltic pump that meters in a sodium bicarbonate solution and deionized distilled water in a ratio of 7.75 +/- 0.07 w/v %.

Once the culture broth is combined into and homogenized using the described operating parameters and under control ranges, reconstituted probiotics are added through the feeding port into the carbohydrate mixture. Fermentation will take 10-20 hours, depending on the strain or strain consortium used in the bioreaction. After that time, the ferment is downloaded as is and placed in a non-translucent glass container. This ferment is referred as inoculum, and at the end of the first bioreaction process will have a concentration of at least 5×10₁₃ to 1×10₄ CFU in total inoculum, and having achieved 2 decimal logarithms of biomass growth. The refrigeration stability of this inoculum will last up to 3 weeks in domestic refrigeration at 5-9 °C.

Simultaneously to inoculum production, it is necessary to prepare 3 more mixtures for the elaboration of 200kg of food in controlled dynamic fermentation for bacteriostasis of beneficial microorganisms, keeping them alive and metabolically active for human consumption. It is important to mention that all of the ingredients listed below must have innocuousness required by ISO Standard 22000:2018 at the very least, since they are food grade. The first mixture is described in Table 4.

**Table 4. First mixture.**

| Number | Ingredient | Amount | Range | Units |
|---|---|---|---|---|
| 1 | Gelli ng agent | 4.800 | +/- 0.240 | Kg |
| 2 | Gum | 0.346 | +/- 0.0173 | Kg |
| 3 | Coco nut flour | 1.730 | +/- 0.835 | Kg |
| 4 | Agav e inulin | 16.700 | +/- 0.835 | Kg |
| TOTAL | | 23.576 | | |
| Solid base | | 22.633 | | |
| Maximum humidity % | | 0.94304 | | |

Where the gelling agent may be carrageenan, gelatin, agar-in all circumstances, agar must have 290 +/-3 degrees Bloom, 5.5 +/-0.5 pH, humidity no higher than 11.00% and 30 sieve mesh according to ASTM (opening of 0.596mm), food grade gum that may be xanthate or guar gum, in any case, with a Brookfield viscosity (1% IN 1% KCL) of 1,580 CP within a range from 1,200 to 1,600 CP, 5.5 +/- 0.5 pH,
humidity no higher than 11.00% and particle size of 30 ASTM (opening of 0.596MM), flour that may be coconut, almond, oatmeal, rye and wheat flour or a mixture of them, which must have 5.7 +/- 0.5 pH, humidity no higher than 5.50 +/- 0.5% and 30 sieve mesh according to ASTM (opening of 0.596mm), agave inulin with 6 +/- 1 pH, relative humidity no higher than 6%, with a concentration of agave fructans of at least 90%, where at least the 80% of them have a molecular structure with beta 2-1 and beta 2-6 binds resistant to hydrolysis by human digestive enzymes, but that they can be fermented by native lactic acid bacteria of the human gastrointestinal tract. The total of this first solid gelling mixture is 23.576 +/- 1.18kg. The total of the first gelling mixture is 23.576 +/- 1.18kg, where the solid base is 22.633 +/-1.1316kg, and the maximum humidity % is 0.94304 +/- 0.0471.

The second mixture is referred as acid and its components in solid base are described in Table 5.

**Table 5. Second mixture.**

| Num ber | Ingredient | Amount | Range | Unit s |
|---|---|---|---|---|
| 1 | Vitamin pre-mixture | 0.39 | +/- 0.0195 | Kg |
| 2 | Dehydrat ed fruit | 8.05 | +/- 0.4025 | Kg |
| 3 | Citric acid | 0.276 | +/- 0.0138 | Kg |
| TOTAL | | 8.716 | | |
| Solid base | | 8.41094 | | |
| Maximum humidity % | | 0.30506 | | |

Where the vitamin pre-mixture has the composition described in Table 6.

**Table 6. Vitamin pre-mixture.**

| Num ber | Ingredient | Amount in 100g | Range | Unit s |
|---|---|---|---|---|
| 1 | Nisin | 8503.81 | +/-850.38 | mg |
| 2 | Ascorbic acid | 47701.00 | +/-4770.10 | mg |
| 3 | Folic acid | 332.71 | +/- 33.27 | mg |
| 4 | Vitamin B12 | 1.54 | +/-0.15 | mg |
| 5 | Vitamin E | 8374.25 | +/-837.43 | mg |
| 6 | Thiamine | 678.11 | +/-67.81 | mg |
| 7 | Riboflavi n | 726.00 | +/- 72.60 | mg |
| 8 | Pyridoxine | 800.74 | +/- 80.07 | mg |
| TOTAL | | 67118.1 7 | | mg |
| | | 67.12 | | g |

Said mixture has a 4.0 +/-1.2 pH and sieve mesh 30 according to ASTM (opening of 0.596Mm), humidity no higher than 5 +/-1%, citric acid with 4 +/-1 pH, percent of water presence of 0.5 +/-0.3, sieve mesh 30 according to ASTM (opening of 0.596mm), natural dehydrated fruit, anyone that meets the following conditions: humidity no higher than 6%, 5 +/-2 pH, apparent density of 0.65 +/-0.06g/cm, sieve mesh 30 according to ASTM (opening of 0.596mm). The total of the second mixture referred as solid acid is of 8.716 +/-0.4358 with a solid base of 8.410 +/-0.4205 and maximum relative humidity % of 0.305 +/-0.0153.

The third mixture is referred as a carbohydrate mixture, and it is a syrup constituted by the ingredients listed in Table 7.

**Table 7. Carbohydrate mixture.**

| Number | Ingredient | Amount | Range | Units |
|---|---|---|---|---|
| 1 | Water | 130 | +/- 0.0325 | L |
| 2 | Agave syrup | 11.5 | +/- 1.15 | Kg |
| 3 | Inulin | 66.7 | +/- 6.67 | Kg |
| | TOTAL | 208.2 | | |
| | Solid base | 69.575 | | |
| | Water presence | 138.625 | | |

Where water has 7 +/-0.5 pH, 95-110ppm of hardness, residual chlorine between and 135ppm, the agave inulin has 6 +/-1 pH, relative humidity no higher than 6%, with a concentration of agave fructans of at least 90%, where at least the 80% of them have a molecular structure of beta 2-1 and beta 2-6 binds, resistant to hydrolysis by human digestive enzymes, but that they can be fermented by lactic acid bacteria of the human gastrointestinal tract, and agave syrup with 5 +/-1 pH, relative humidity of 25% +/-1% and a composition of carbohydrates according to Table 8.

**Table 8. Carbohydrate composition.**

| | |
|---|---|
| Fructose (%) | 84.0 min. |
| Glucose (%) | 13.0 max. |
| Sucrose (%) | 4.0 max. |
| Inulin - Fructooligosaccharid es (%) | 0.50 - 3.0 |
| Mannitol (%) | 0.50 max. |
| Other sugars (%) | 2.0 max. |

The third mixture total referred as carbohydrates in solid base is 69.575 +/-6.9575Kg, with presence of water of 138.625 +/-0.895L, for a total of carbohydrate syrup of 208.2 +/-7.8525Kg.

Mixture total to be processed is shown in Table 9 below.

**Table 9. Total mixtures of processed food.**

| Num ber | Ingredient | Amount | Range | Units |
|---|---|---|---|---|
| 1 | Gelling agent | 23.576 | +/- 1.1788 | Kg |
| 2 | Acid mixture | 8.716 | +/- 0.4358 | Kg |
| 3 | Carbohyd rate syrup | 208.2 | +/- 7.8525 | L |
| TOTAL | | 240.492 | | |
| Solid base | | 100.619 | | |
| Water presence | | 139.8731 | | |

Total mixtures in solid base is of 100.619 +/-8.56096Kg, where water presence is of 139.8731 +/-0.9574L, to produce a total of 240.492 +/-9.4671Kg.

Once we have thoroughly described the mixtures, the key characteristics of food grade supplies, their solid bases and water presence, we will define in detail the process to obtain a food in controlled dynamic fermentation for bacteriostasis of beneficial microorganisms, by keeping them alive and metabolically active for human consumption, with no limitation to animal consumption.

Using a 250L pot with automatic temperature control, it is proceeded to prepare the carbohydrate syrup mixture. First, 130 +/-0.0325L of water are poured at 85 +/-2° C, agitation is started preferably using a vertical motor at a speed of 1,000 +/-400 revolutions per minute (RPM), 11.5 +/-1.15Kg of agave syrup are then combined into by stirring for 10 minutes. Afterwards, 66.7 +/-6.67Kg of agave inulin are added in the solid base by stirring for 25 minutes. While the original solid base of this carbohydrate syrup mixture is of 69.575 +/-6.9575Kg and the presence of water is 138.625 +/-0.895, achieving a ratio of 1:1.99 (solids-water presence), after stirring for 85 minutes at 85 +/-2° C, the mixture varies its total solids and water presence ratio to 1:1.88, as a result of evaporation 7.625 +/-0.0457L. This mixture achieves a 6.5 +/-0.5 pH, with a total of 69.575 +/-6.9575Kg in solid base plus 131.00 +/-0.845 of water presence, totalizing 200.575 +/-0.8914Kg.

Once the carbohydrate syrup mixture is homogenized, temperature is increased to 95 +/-2° C, and speed up to 1,200 +/-400 rpm with 6.5 +/-0.5 pH in order to incorporate the entire gelling mixture, emptying the pot feeding hopper, whilst stirring. This will increase the solid base in 8.410 +/-0.4205Kg and will modify the water presence in a range that is not greater than 0.305 +/-0.0153. Stirring and temperature are maintained for 25 +/-3 minutes; after that time, the resulting mixture will be a homogenized gelling mixture of 204.19 +/-10.2095Kg, where the solid base is 77.985 +/-7.377 and the water presence will decrease to 126.205 +/-0.5048L, as a result of water evaporation by 5 +/-0.020L, producing a (solids-water presence) ratio of 1:1.61. The mixture pH will be of 6.0 +/-0.5.

For the final addition of the acid mixture, temperature is reduced to 60 +/-3° C and agitation speed to 900 +/-100rpm. It is then poured into the feeding hopper of the pot. The acid mixture total is 8.716 +/-0.4358Kg, keeping agitation for 20 minutes more. After that time, an acidified homogenized mixture is obtained with 5.2 +/-0.3 pH, a total solid base of 86.3959 +/-7.802Kg and presence of water with a decrease of 3 +/-0.012L due to evaporation during this phase of the process, reaching at 123.205 +/-0.519L. The resulting product is a food of 209.600 +/-8.321Kg.

In the pot, agitation speed is reduced to 700 +/-50 rpm so as to decrease temperature in the produced food to 37.5 +/-5° C. This is achieved using a temperature control loop that triggers the access of water cooler at 6 +/-1 ° C, feeding the pot jacket for approximately 20 minutes. At this moment, the food to be fermented with the inoculum should have the following physical, chemical, nutritional and biological characteristics, as shown in Table 10.

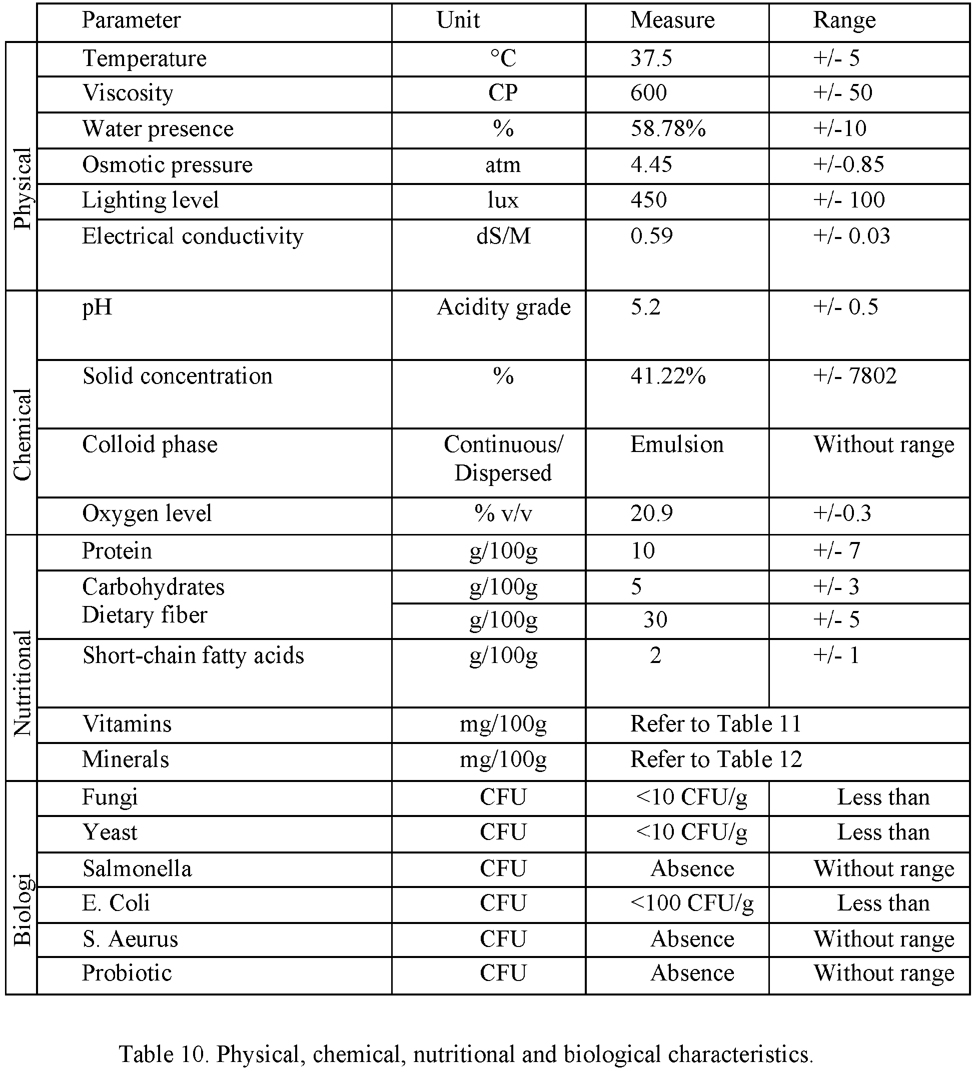

**Table 11. Vitamin content in 100g of processed food.**

| Ite m | Ingredient | Amoun t in 100g | Range | Units |
|---|---|---|---|---|
| 1 | Nisin | 8.50 | +/-1,276 | mg |
| 2 | Ascorbic acid | 47.70 | +/-7.155 | mg |
| 3 | Folic acid | 0.33 | +/-0.050 | mg |
| 4 | Vitamin B12 | 0.0015 | +/-0.0002 | mg |
| 5 | Vitamin E | 8.37 | +/-1.256 | mg |
| 6 | Thiamine | 0.68 | +/-0.102 | mg |
| 7 | Riboflavin | 0.73 | +/-0.109 | mg |
| 8 | Pyridoxine | 0.80 | +/-0.120 | mg |

**Table 12. Content in 100g of processed food.**

| I t e m | Ingredient | Amount in 100g | Range | Units |
|---|---|---|---|---|
| 1 | Calcium | 1.41 | +/-0.211 | mcg |
| 2 | Magnesium | 2.32 | +/-0.348 | mcg |
| 3 | Potassium | 10.47 | +/-1.570 | mcg |
| 4 | Phosphorus | 11.14 | +/-1.672 | mcg |
| 5 | Iron | 0.62 | +/-0.093 | mcg |
| 6 | Zinc | 0.55 | +/-0.082 | mcg |

A homogeneous nutritional composition is achieved following the sequence of ingredients, temperatures, speeds and stirring times defined. Failure to follow these parameters described will result in deficiencies in integration, lumps, solid sedimentation and aggregated areas with different physical and chemical parameters.

It is imperative that the processed food be homogeneous in any and all of the physical, chemical, nutritional and biological safety parameters as described in Table 10, before proceeding with inoculation.

It is necessary to precondition the inoculum at a room temperature of 25 +/-3°C for at least 10 minutes; therefore, if the inoculum is refrigerated, you should consider the time it takes to reach room temperature.

The inoculum has a total weight of 7.09 +/-0.6326Kg in liquid form, and UCF concentration of at least 5×10₁₃ to 1×10₁₄ CFU in the total inoculum.

The inoculation process follows this sequence: first, you should completely stop mixing the already homogeneous processed food, and then pour evenly 3.5 +/-0.25Kg of inoculum into the pot surface where the processed food has been prepared according to the parameters described in Table 10.

To integrate the inoculum homogeneously after pouring the total 3.5 +/-0.25Kg of inoculum into the 240.50 +/-9.45Kg of processed food, according to the parameters described in Table 10, it is then stirred using a spiral blade making eights at 100 +/-20 revolutions per minute. This is done to prevent air from entering. Continue stirring for 4 +/-1 minutes to obtain a food with fermentation process of 243.750 +/-9.70Kg.

Fermented processed food will have a concentration of about 2.5×10₁₃ to 5×10₁₃ CFU which, in turn, cause a concentration from 1.03×10₈ to 2.05×10₈ CFU/g of fermented processed food.

The homogeneous fermented food must be left at rest for 25 +/-5 minutes at 37.5 +/-5°C.

Packing is recommended at a temperature no cooler than 35°C and not exceeding 40°C to allow it to circulate through the injectors of the different available packing machines, such as sachets, pouches or jars, in every case complying with oxygen barrier, since probiotics are facultative or strict anaerobic bacteria.

Fermented product could be conditioned at 25 +/-3°C.

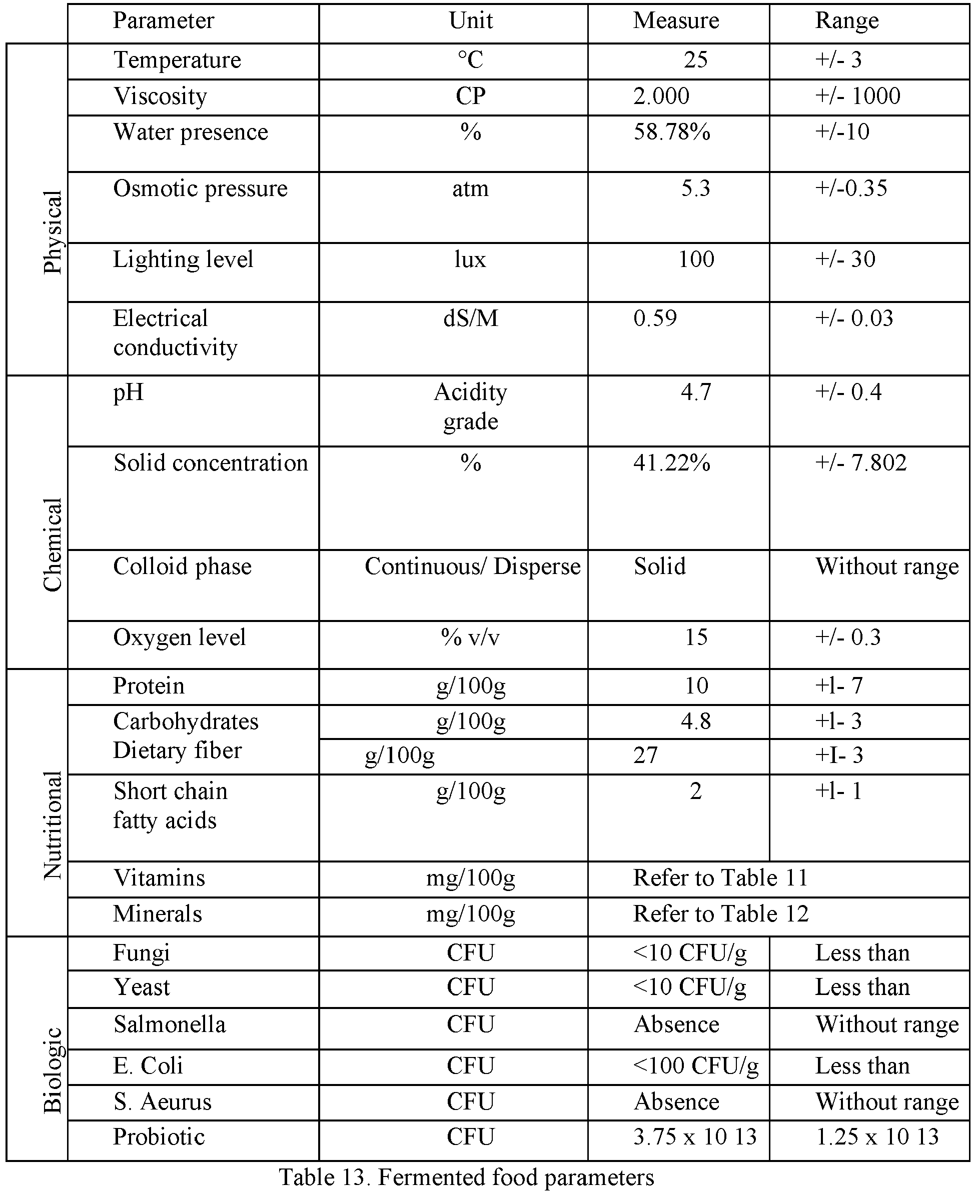

Table 13 shows physical, chemical, nutritional and biological parameters under these conditions if, and only if, the patent's sequence is completely followed by, which ensures innocuousness, biological integrity and sustainability of probiotic bacteria that will generate secondary metabolites such as lactic, propionic and butyric acids, proteins like pyridoxine, as well as some lipid and/or protein enzymes. These secondary metabolites and the fermented food gelation are responsible for parameters change in Tables 10-13.

Table 13 parameters allow for an initial bacteriostasis that will be dynamic, gradually increasing as temperature goes from 28°C to 22°C, with no limitation to lower temperatures and domestic freezing up to -10°C.

The superiority of the art described herein lies in that, even being out of the freezer at room temperature, the bacteriostasis will allow for a stability time in the fermented food of up to 8 months, keeping innocuousness and being clear and free from pathogenic microorganisms, with a probiotic load that, at most, will be degraded to no more than a logarithm with base 10, that is, keeping content between 1.03×10₇ and 2.05×10₇ CFU/g of
fermented food.

Even organoleptic conditions will only vary in terms of taste, being slightly more acid, reaching a pH level of 4.0 maximum.

Physical, chemical, nutritional and biological parameters after 8 months of stability at room temperature of 25 +/-3°C will have slight variations, but throughout that period, they will have allowed for a stress state where probiotic bacteria are far from the osmotic pressure parameters responsible for turgor pressure or plasmolysis. Likewise, acidity will be increased due to dynamic fermentation of the probiotic bacteria, with a minimum metabolic activity level, but being alive and active for their stability. Vitamins and minerals are significantly important, as well as substrates in carbohydrates as a source of energy, and proteins, which are necessary for nitrogen compounds in a level that would not create any risk for the human consumer's health, who could eat the fermented food along the entire shelf life, that is, the 8 months complying with stability requirement.

Physical, chemical, nutritional and biological parameters of the fermented foods are described after 8 months of stability, which represent the biological threshold after which probiotic bacteria start having viability problems due to acidity, lack of carbohydrate substrates, proteins and osmotic pressure modification, as a result of acidification and solid substrates decrease.

Bacteriostasis is lost and, as a consequence, probiotic bacteria will tend to decrease rapidly; their cells will be degraded biologically and, if some spores inhibited by yeast are latent, mainly the fermented food, they will also tend to lose innocuousness.

Under organoleptic conditions, the fermented food will lose gelified viscosity and will have an unpleasant acid taste, far less sweet. It is worth mentioning that room temperature is a great parameter for growth and development of yeasts, which will still find an environment with nutrients.

Bacteriostasis is a fine balance among any and all of the physical, chemical, nutritional and biological parameters described in Table 13. Considering the upper and lower ranges of each parameter, this spectrum of variables produces a stable bacteriostasis within a range where probiotics will maintain a minimum metabolic activity. By keeping them alive and active, this condition is vitally important since the fermented food allows for gastrointestinal tract colonization from the first third of the small intestine, and the fermented foods passes through without causing damages to inoculated and already stressed bacteria. It is important to highlight that the most
aggressive conditions of the gastrointestinal tract are similar to the stress caused by bacteriostasis.

Table 14 describes threshold conditions of bacteriostasis after 8 months.

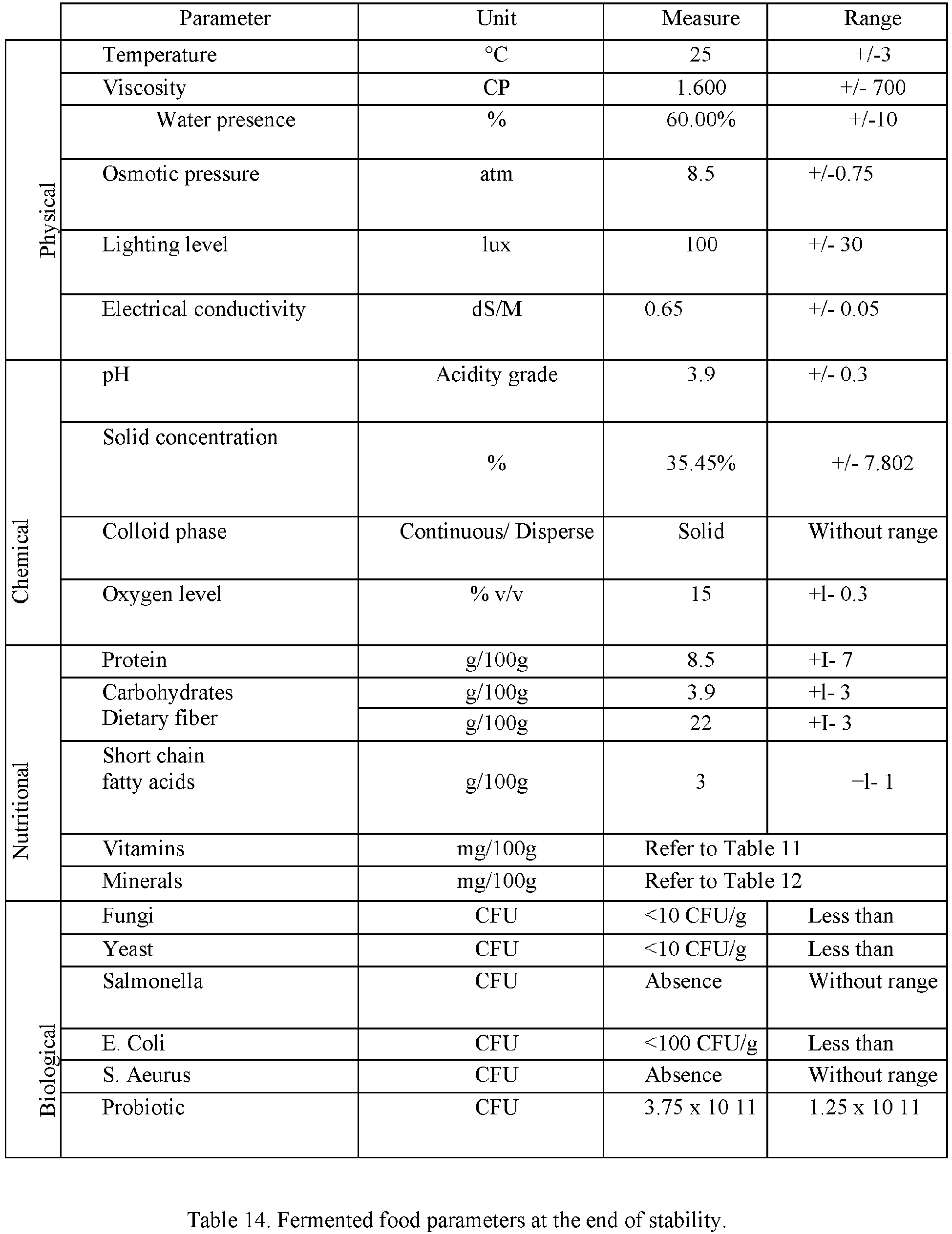

After thoroughly describing the process to obtain bacteriostasis from a controlled dynamic fermented food, by keeping microorganisms alive and metabolically active for human consumption and, with that, the benefits of both the microorganisms and the food matrix nutrients to be fermented and secondary metabolites, proteins, enzymes and biomass provide to the health, nutrition and wellness of the person who consumes the ferment with bacteriostasis, there is a description of three examples of application of this innovative biotechnology in fermented foods.

### Example 1: Method to obtain a controlled and dynamic fermented product based on pineapple and coconut, with Lactobacillus Rhamanosus and Bifidus bacterium Lactis bacteriostasis.

1. Prepare a solid mixture with:

| | | |
|---|---|---|
| Gelatin | 4.00 +/-0.250kg | |
| Xanthan gum | 346 +/-5g Coconut flour | 1.73 +/-0.100kg |

Preferably in a pant type mixer for 5 +/-0.5 minutes.
2. Pour into a food grade container 16.7 +/-0.150kg of agave inulin with a molecular structure with beta 2-1 and beta 2-6 binds, resistant to hydrolysis by human digestive enzymes.
3. In a 250 +/-0.200kg pot, pour into 15 +/- 0.250L of water at 80 +/-5°C.
4. Add 16.7 +/-0. 150kg of agave inulin into the pot, with a molecular structure with beta 2-1 and beta 2-6 binds, resistant to hydrolysis by human digestive enzymes.
5. Stir for 6 +/-0.5 minutes at 1300 +/-200 revolutions per minute.
6. Pour 10 +/-0.500L of water into the pot at 80 +/-5°C.
7. Add 5.75 +/-0.250kg of agave syrup into the pot mixture.
8. Pour 5 +/-0. 150L of water into the pot at 80 +/- 5°C.
9. Stir for 4 +/-0.5 minutes at 1300 +/-200 revolutions per minute.
10. Pour another 10 +/-0.500L of water into the pot at 80 +/- 5°C.
11. Verify that the temperature of the syrup obtained in the mixture is between 55 and 65°C maximum.
12. Add the mixture of point 1 into the pot.
13. Stir for 5 +/-0.5 minutes at 1300 +/-200 revolutions per minute.
14. Dissolve 138 +/-10g of citric acid plus 195 +/-50g of vitamin pre-mixture into 2 +/-0.100L of water.
15. Add the mixture of point 14 into the pot with 4.0 +/-0.50kg of dehydrated pineapple.
16. Stir for 8 +/-0.5 minutes at 1300 +/-200 revolutions per minute. 17. Pour 6 +/-0.500L of water into the pot at 25 +/-5°C.
17. Pour 6 +/-0.200L of additional water into the pot at 25 +/-5°C.
18. Stir for 6 +/-0.5 minutes at 1300 +/-200 revolutions per minute. Check that final temperature of the mixture is between 35 and 40°C.
19. Once homogenized, the food matrix is inoculated without stirring with 1 +/-0.150L of ferment and CFU concentration of at least 5×10₁ to 1×10₁₄ CFU of the preparation as described in the Detailed description of the invention section in this patent. For these examples, strains Lactobacillus Rhamnosus NH001 and Bifidus Bacterium Bio 07 were the synergistic strains used to cultivate in the bioreaction.
20. By stirring using an eight-shaped pattern so as to prevent air injection, or using a vacuum pot, mix at 30-60 revolutions per minute for 1 +/-0.2 minutes.
21. Leave the product at rest for a first controlled fermentation of the food during 25 +/-3 minutes.
22. Proceed with packaging, in containers or sachets. In every case, the package should have an oxygen barrier, and the appropriate seal ensuring this condition, and a color layer to protect the food in dynamic fermentation from sunlight.
23. Once the food is packed at room temperature and with nonsporeforming microorganisms, it could be stored at room temperature for up to 8 months.

### Example 2.- Method to obtain a controlled and dynamic fermented product based on mango and coconut with Lactobacillus Acidophilus NCFU and Bifidus bacterium Lactis bacteriostasis.

1. Prepare a solid mixture with:

| | |
|---|---|
| Carrageenan | 2.00 +/-0.125kg |
| Pectin | 2.00 +/-0.125kg. |
| Xanthan gum | 346 +/-5g |
| Coconut flour | 1.73 +/-0.100kg |

Preferably in a pant type mixer for 5 +/-0.5 minutes.
2. Pour into a food grade container 16.7 +/-0.150kg of agave inulin with a molecular structure of beta 2-1 and beta 2-6 binds resistant to hydrolysis by human digestive enzymes.
3. In a 250 +/-0.200kg pot, pour into 15 +/-0.250L of water at 80 +/-5°C.
4. Add into the pot 16.7 +/-0.150kg of agave inulin with a molecular structure of beta 2-1 and beta 2-6 binds resistant to hydrolysis by human digestive enzymes.
5. Stir for 6 +/-0.5 minutes at 1300 +/-200 revolutions per minute.
6. Pour 10 +/-0.500L of additional water into the pot at 80 +/-5°C.
7. Add 5.75 +/-0.250kg of agave syrup into the pot mixture.
8. Pour another 10 +/-0.500L of water into the pot at 80 +/- 5°C.
9. Stir for 4 +/-0.5 minutes at 1300 +/-200 revolutions per minute.
10. Pour 10 +/-0.500L of water into the pot at 80 +/-5°C.
11. Verify that the temperature of the syrup obtained in the mixture is between 55 and 65°C maximum.
12. Add the mixture of point 1 into the pot.
13. Stir for 5 +/-0.5 minutes at 1300 +/-200 revolutions per minute.
14. Dissolve 138 +/-10g of citric acid plus 195 +/-50g of vitamin pre-mixture into 2 +/-0.100L of water.
15. Add the mixture of point 14 into the pot with 6.5 +/-0.50kg of dehydrated mango.
16. Stir for 8 +/-0.5 minutes at 1300 +/-200 revolutions per minute.
17. Pour 6 +/-0.200L of water into the pot at 25 +/- 5°C.
18. Stir for 6 +/-0.5 minutes at 1300 +/-200 revolutions per minute. Check that final temperature of the mixture is between 35 and 40°C.
19. Once homogenized, the food matrix is inoculated without stirring with 1 +/-0.150L of ferment and concentration of at least 5×10₁₃ to 1×10₁₄ CFU of the preparation as described in the Detailed description of the invention section in this patent. For these examples, strains Lactobacillus NCFM and Bifidus Bacterium Bio-07 were the synergistic strains used to cultivate in the bioreaction.
20. By stirring using an eight-shaped pattern so as to prevent air injection, or using a vacuum pot, mix at 30-60 revolutions per minute for 1 +/-0.2 minutes.
21. Leave the product at rest for a first controlled fermentation of the food during 25 +/-3 minutes.
22. Proceed with packaging, in containers or sachets. In every case, the package should have an oxygen barrier, and the appropriate seal ensuring this condition, and a color layer to protect the food in dynamic fermentation from sunlight.
23. Once the food is packed at room temperature and with nonsporeforming microorganisms, it could be stored at room temperature for up to 8 months.

### Example 3: Method to obtain a controlled and dynamic fermented product based on strawberry and coconut with a consortium of Lactobacillus Plantarum

1. Prepare a solid mixture with:

| | | |
|---|---|---|
| Carrageenan | 2.00 +/-0.250kg | |
| Pectin | 2.00 +/- 0.125kg. | |
| Gelatin | 0.50 +/- 0.010kg. Coconut flour | 1.73 +/-0.100kg |

Preferably in a pant type mixer for 5 +/-0.5 minutes.
2. Pour into a food grade container 16.7 +/-0.150kg of agave inulin with a molecular structure with beta 2-1 and beta 2-6 binds, resistant to hydrolysis by human digestive enzymes.
3. In a 250 +/-0.200kg pot, pour into 15 +/-0.250L of water at 80 +/-5°C.
4. Add into the pot 16.7 +/-0.150kg of agave inulin with a molecular structure of beta 2-1 and beta 2-6 binds resistant to hydrolysis by human digestive tract.
5. Stir for 6 +/-0.5 minutes at 1300 +/-200 revolutions per minute.
6. Pour 10 +/-0.500L of water into the pot at 80 +/- 5°C.
7. Add 5.75 +/-0.250kg of agave syrup into the pot mixture.
8. Pour another 10 +/-0.500L of water into the pot at 80 +/- 5°C.
9. Stir for 4 +/-0.5 minutes at 1300 +/-200 revolutions per minute.
10. Pour 10 +/-0.500L of additional water into the pot at 80 +/-5°C.
11. Verify that the temperature of the syrup obtained in the mixture is between 55 and 65°C maximum.
12. Add the mixture of point 1 into the pot.
13. Stir for 5 +/-0.5 minutes at 1300 +/-200 revolutions per minute.
14. Dissolve 138 +/-10g of citric acid plus 195 +/-50g of vitamin pre-mixture into 2 +/-0.100L of water.
15. Add the mixture of point 14 into the pot with 5.8 +/-0.50kg of dehydrated strawberry.
16. Stir for 8 +/-0.5 minutes at 1300 +/-200 revolutions per minute.
17. Pour another 10 +/-0.500L of coconut water into the pot at 25 +/- 5°C.
18. Stir for 6 +/-0.5 minutes at 1300 +/-200 revolutions per minute. Check that final temperature of the mixture is between 35 and 40°C.
19. Once homogenized, the food matrix is inoculated without stirring with 1 +/-0.150L of ferment and concentration of at least 5×10₁₃ to 1×10₁₄ CFU of the preparation as described in the Detailed description of the invention section in this patent. For these examples, strains are a consortium of Lactobacillus Plantarum, synergistic strains to be cultivated in the bioreaction.
20. By stirring using an eight-shaped pattern so as to prevent air injection, o using a vacuum pot, mix at 30-60 per minute for 1 +/-0.2 minutes.
21. Leave the product at rest for a first controlled fermentation of the food during 25 +/-3 minutes.
22. Proceed with packaging, in containers or sachets. In every case, the package should have an oxygen barrier, and the appropriate seal ensuring this condition, and a color layer to protect the food in dynamic fermentation from sunlight.
23. Once the food is packed at room temperature and with nonsporeforming microorganisms, it could be stored at room temperature for up to 8 months.

## Claims

1. A food in controlled dynamic fermentation for generating bacteriostasis in alive and metabolically active microorganisms, allowing for stable shelf life at room temperature for up to 8 months.

2. The food in controlled dynamic fermentation to generate bacteriostasis according to claim 1, where the first fermentation contains agave inulin with a molecular structure with beta 2-1 and beta 2-6 binds, resistant to hydrolysis by human digestive enzymes, but that they can be fermented by native lactic acid bacteria from the human gastrointestinal tract.

3. The food in controlled dynamic fermentation to generate bacteriostasis according to claim 1 and 2, where the first fermentation has a protein with the following profile in its aminogram:
| Amino acids | Mg/L | Range |
|---|---|---|
| Isoleucine | 5.30 | +/- 0.795 |
| Leucine | 8.70 | +/- 1305 |
| Lysine | 5.70 | +/- 0.855 |
| Methionine and cysteine | 3.30 | +/- 0.495 |
| Phenylalanine and tyrosine | 19.00 | +/- 2.850 |
| Threonine | 4.00 | +/- 0600 |
| Tryptophan | 0.60 | +/- 0.090 |
| Valine | 5.10 | +/- 0.765 |

4. The food in controlled dynamic fermentation to obtain bacteriostasis according to claim 3, where the first fermentation includes a yeast extract, typically Saccharomyces cerevisiae or any other used in the bakery or brewing industry, and food grade salt composition with the following profile:
| Salt (food grade) | % |
|---|---|
| Sodium acetate, anhydrous | 54.35 % |
| Potassium phosphate, dibasic | 21.74 % |
| Ammonium Citrate, dibasic | 21.74 % |
| Magnesium sulfate | 2.17% |
| TOTAL | 100.00 % |

5. The food in controlled dynamic fermentation to obtain bacteriostasis according to claim 4, where the control parameters of the bioreaction are the following:
| Operati on paramet ers | Unit | Magnit ude | Operatio n range |
|---|---|---|---|
| Pressure | Lb./in² | 15 | +/-2 |
| Agitatio n Speed | RPM | 90 | +/- 10 |
| Temperat ure | °C | 37.5 | +/-2 |
| pH | 0-14 scale | 5.7 | +/- 0.4 |
| Time | Hours | 15 | +/-s |

6. The food in controlled dynamic fermentation to obtain bacteriostasis according to Claim 5, where the first fermentation results in an inoculum that will have a CFU concentration of at least 5×10₃ to 1× 10₁₄ CFU in the total inoculum, achieving 2 decimal logarithm of biomass growth.

7. The food in controlled dynamic fermentation to obtain bacteriostasis according to Claim 6, which is inoculated in a food that requires 3 mixtures for preparing a total of 200kg, where the first mixture is characterized because it contains the following:
| Numb er | Ingredient | Amount | Range | Units |
|---|---|---|---|---|
| 1 | Gelling agent | 4.800 | +/- 0.240 | Kg |
| 2 | Gum | 0.346 | +/- 0.0173 | Kg |
| 3 | Coconut flour | 1730 | +/- 0.0865 | Kg |
| 4 | Agave inulin | 16.700 | +/- 0.835 | Kg |
| | TOTAL | 23.576 | | |
| | Solid base | 22.633 | | |
| | Maximum humidity % | 0.94304 | | |

8. The food in controlled dynamic fermentation to obtain bacteriostasis according to Claim 7, where the second of 3 mixtures necessary to prepare 200Kg of food to inoculate is characterized because it contains the following:
| Num ber | Ingredient | Amount | Range |
|---|---|---|---|
| 1 | Vitamin pre-mixture | 0.39 | +/- 0.0195 |
| 2 | Dehydrated fruit | 8.05 | +/- 0.4025 |
| 3 | Citric acid | 0.276 | +/- 0.0138 |
| | TOTAL | 8.716 | |
| | Solid base | 8.41094 | |
| | Maximum humidity % | 0.30506 | |

9. The food in controlled dynamic fermentation to obtain bacteriostasis according to Claim 8, where the second of 3 mixtures necessary to prepare 200kg of food to inoculate is characterized because it contains a vitamin complement with the following characteristics:
| Nu mbe r | Ingredient | Amount in 100g | Range | Units |
|---|---|---|---|---|
| 1 | Nisin | 8503.81 | +/- 850.38 | mg |
| 2 | Ascorbic acid | 47701.00 | +/- 4770.10 | mg |
| 3 | Folic acid | 332.71 | +/- 33.27 | mg |
| 4 | Vitamin B12 | 1.54 | +/-0.15 | mg |
| 5 | Vitamin E | 8374.25 | +/- 837.43 | mg |
| 6 | Thiamine | 678.11 | *+/-67.81 | mg |
| 7 | Riboflavin | 726.00 | +/- 72.60 | mg |
| 8 | Pyridoxine | 800.74 | +/- 80.07 | mg |
| | TOTAL | 67118.17 | | mg |
| | | 67.12 | | g |

10. The food in controlled dynamic fermentation to obtain bacteriostasis according to Claim 9, where the third mixture necessary to prepare 200kg of food to inoculate is a syrup characterized because it contains the following:
| Nu mbe r | Ingredient | Amount | Range | Units |
|---|---|---|---|---|
| 1 | Water | 130 | +/- 0.0325 | L |
| 2 | Carbohydrate syrup | 11.5 | +/- 1.15 | Kg |
| 3 | Inulin | 66.7 | +/- 6.67 | Kg |
| | TOTAL | 208.2 | | |
| | Solid base | 69.575 | | |
| | Water presence | 138.625 | | |

11. The food in controlled dynamic fermentation to obtain bacteriostasis according to Claim 10, where the total 200kg of food to inoculate have the following physical, chemical, nutritional and biological parameters:

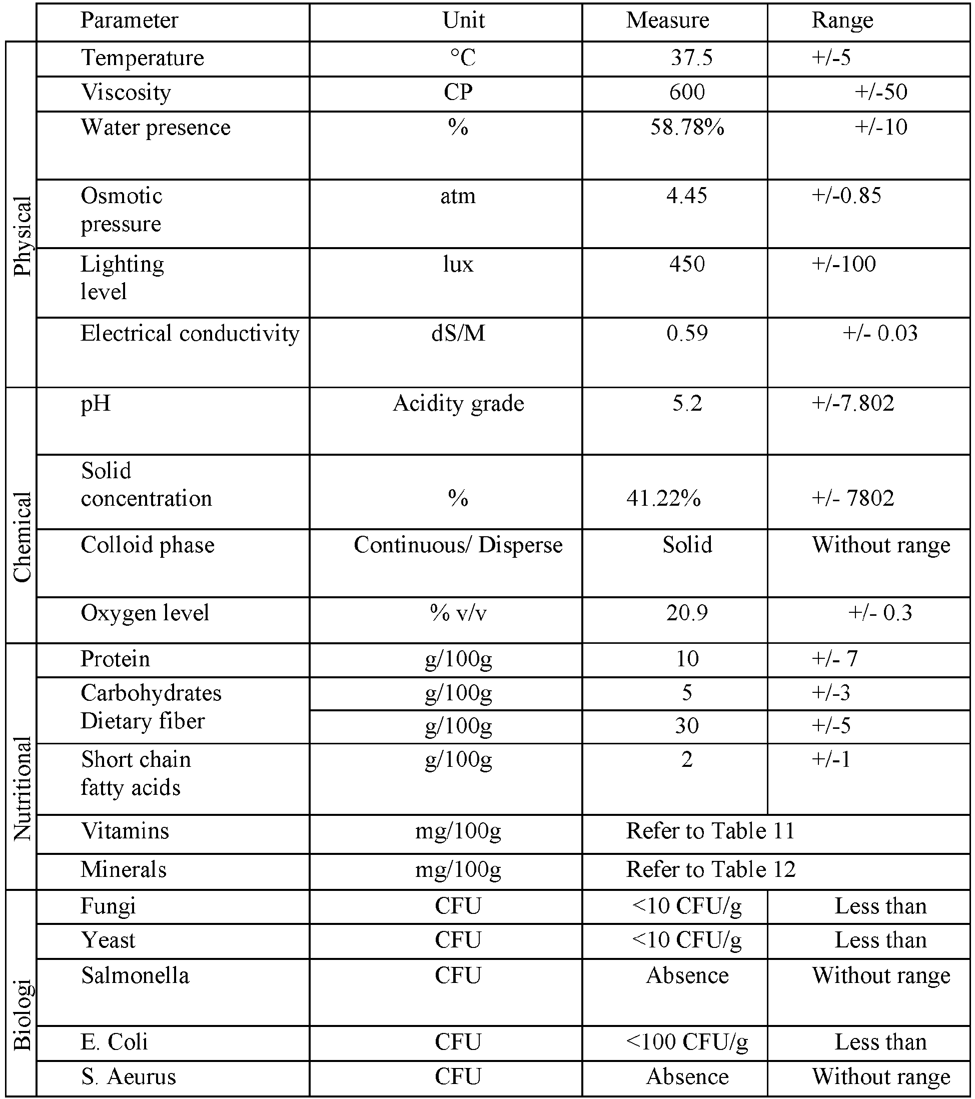

12. The food in controlled dynamic fermentation to obtain bacteriostasis according to Claim 11, where the total 200kg of food to inoculate at 25 +/-5°C have the following physical, chemical, nutritional and biological parameters: 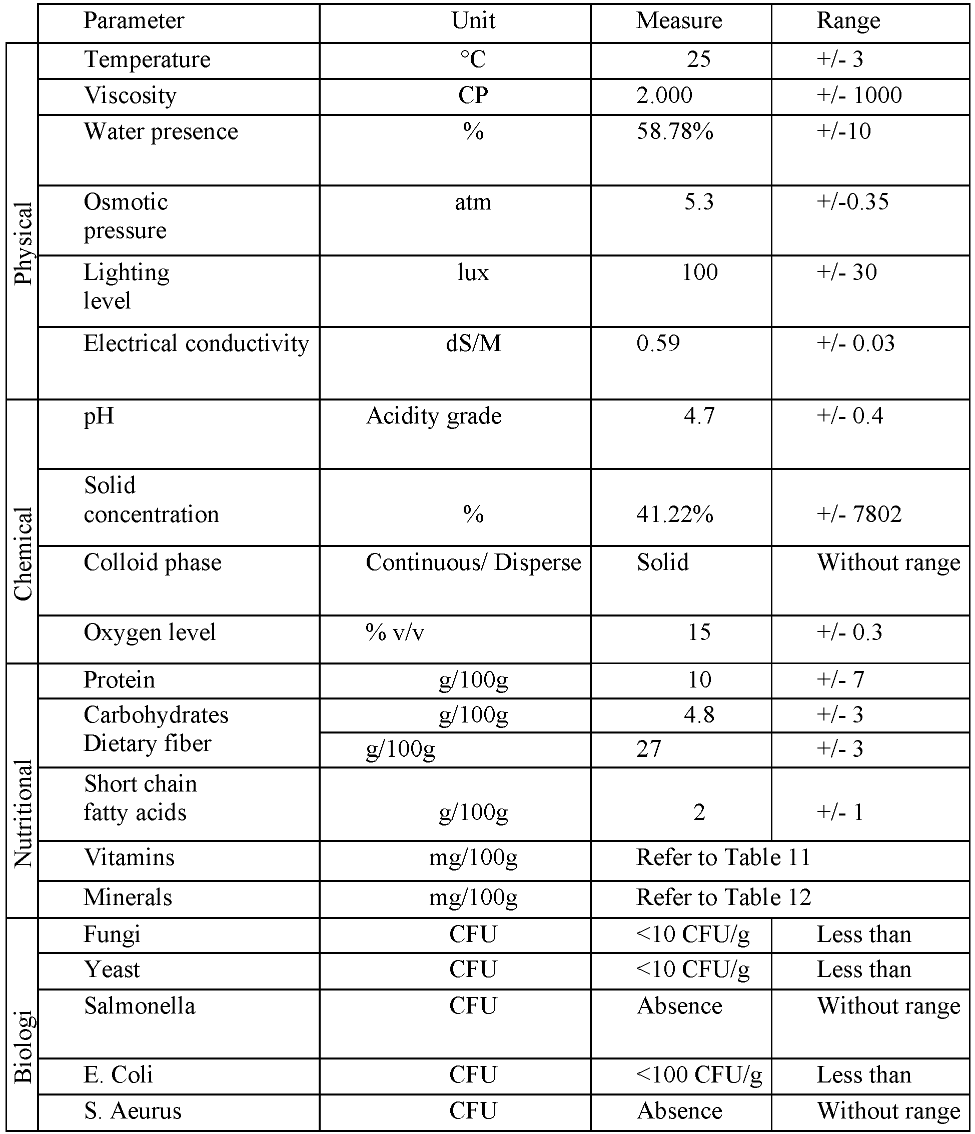 where the convergence of any and all of the parameters described generates bacteriostasis producing a controlled and dynamic fermentation in the food at room temperature, free from pathogenic microorganisms, and keeping innocuousness and sensory characteristics.

13. The food in controlled dynamic fermentation to obtain bacteriostasis according to Claim 12, where even after 8 months, physical, chemical, nutritional and biological parameters are the following:
